# EUROPEAN PATENT APPLICATION

(11) **EP 3 031 392 A1**
(43) Date of publication of application: **15.06.2016**
(21) Application number: 15195418.7
(22) Date of filing: 19.11.2015
(51) Int. Cl.: A61B 5/024, A61B 5/00

(54) **IMPROVING HEART RATE MEASUREMENT**

(30) Priority: 12.12.2014 US 201414569054
(71) Applicant: Polar Electro Oy, 90440 Kempele (FI)
(72) Inventor: Jaatinen, Jukka, 90440 Kempele (FI)
(74) Representative: Kolster Oy Ab

(57) **Abstract**

There is provided a method comprising: obtaining, by an apparatus, heart rate information measured from a user performing a physical activity and user specific information characterizing the user, determining that the heart rate information comprises an interruption period, and generating, based at least on the measured heart rate information and the user specific information, a heart rate change estimation, wherein the heart rate change estimation represents an estimated change in heart rate of the user during the interruption period.

## Description

### FIELD OF THE INVENTION

The invention relates generally to measuring heart activity. More practically, the present invention relates to producing heart rate information.

### BACKGROUND OF THE INVENTION

Measuring heart rate has become more and more popular among users. Thus, it may be beneficial to provide solutions to improve the effectiveness of the measurement.

### BRIEF DESCRIPTION OF THE INVENTION

According to an aspect, there is provided a method comprising: obtaining, by an apparatus, heart rate information measured from a user performing a physical activity and user specific information characterizing the user, determining that the heart rate information comprises an interruption period, and generating, based at least on the measured heart rate information and the user specific information, a heart rate change estimation, wherein the heart rate change estimation represents an estimated change in heart rate of the user during the interruption period.

According to an aspect, there is provided an apparatus comprising: at least one processor and at least one memory including a computer program code, wherein the at least one memory and the computer program code are configured, with the at least one processor, to cause the apparatus to perform operations comprising: obtaining heart rate information measured from a user performing a physical activity and user specific information characterizing the user, determining that the heart rate information comprises an interruption period, and generating, based at least on the measured heart rate information and the user specific information, a heart rate change estimation, wherein the heart rate change estimation represents an estimated change in heart rate of the user during the interruption period.

In an embodiment, the user specific information comprises user parameters comprising at least one physiological attribute of the user.

In an embodiment, the user specific information comprises exertion history data that is characteristics to the user characterized by the user parameters, and wherein the generating of the heart rate change estimation is further based on the exertion history data.

In an embodiment the exertion history data comprises at least some exertion history data of the user.

In an embodiment the exertion history data comprises exertion history data of at least one other user, wherein the exertion history data of the at least one other user is obtained from a training database.

In an embodiment the exertion history data comprises at least one exertion history file comprising at least one of the following: heart rate during the at least one exertion history file; sport of the at least one exertion history file; and environmental conditions of the at least one exertion history file.

In an embodiment the at least one memory and the computer program code are configured, with the at least one processor, to cause the apparatus further to perform operations comprising: obtaining physical activity information of the user during the physical activity, wherein the generating the heart rate change estimation is further based on the physical activity information.

In an embodiment the heart rate change estimation comprises at least one heart rate zone, wherein the estimated change in heart rate of the user during the interruption period is represented with the at least one heart rate zone, and wherein the at least one memory and the computer program code are configured, with the at least one processor, to cause the apparatus further to perform operations comprising: searching a measured heart rate value within the at least one heart rate zone.

In an embodiment, the heart rate change estimation comprises at least one estimated heart rate value.

In an embodiment, the at least one memory and the computer program code are configured, with the at least one processor, to cause the apparatus further to perform operations comprising: reconstructing a heart rate curve on the basis of the heart rate change estimation, wherein the heart rate curve represents changes in heart rate during the interruption period.

In an embodiment, the measured heart rate information comprises at least two heart rate measurements, and wherein the interruption period is determined to be between the said at least two heart rate measurements.

In an embodiment, the at least one memory and the computer program code are configured, with the at least one processor, to cause the apparatus further to perform operations comprising: generating energy expenditure information based on the heart rate change estimation.

In an embodiment, the at least one memory and the computer program code are configured, with the at least one processor, to cause the apparatus further to perform operations comprising: generating an exertion parameter based on the heart rate change estimation, wherein the exertion parameter illustrates effectiveness of the physical exercise.

In an embodiment, the at least one memory and the computer program code are configured, with the at least one processor, to cause the apparatus further to perform operations comprising: displaying at least one of the following during the physical exercise: the energy expenditure information and the exertion parameter.

According to an aspect, there is provided a computer program product embodied on a distribution medium readable by a computer and comprising program instructions which, when loaded into an apparatus, execute a computer process comprising: obtain heart rate information measured from a user performing a physical activity and user specific information characterizing the user, determine that the heart rate information comprises an interruption period, and generate, based at least on the measured heart rate information and the user specific information, a heart rate change estimation, wherein the heart rate change estimation represents an estimated change in heart rate of the user during the interruption period.

According to an aspect, there is provided the subject-matter of the independent claims. Some embodiments are defined in the dependent claims.

One or more examples of implementations are set forth in more detail in the accompanying drawings and the description below. Other features will be apparent from the description and drawings, and from the claims.

### BRIEF DESCRIPTION OF THE DRAWINGS

Embodiments of the present invention are described below, by way of example only, with reference to the accompanying drawings, in which
Figure 1 illustrates a heart activity measurement system to which embodiments of the invention may be applied;
Figure 2 illustrates flow diagram according to an embodiment of the invention;
Figure 3 illustrates an embodiment of the invention;
Figure 4 illustrates an embodiment of the invention;
Figures 5A to 5E illustrate some embodiments of the invention;
Figures 6A to 6B illustrate some embodiments of the invention;
Figure 7 illustrates an embodiment of the invention;
Figure 8 illustrates an embodiment of the invention; and
Figure 9 illustrates a block diagram of an apparatus according to an embodiment of the invention.

### DETAILED DESCRIPTION OF THE INVENTION

The following embodiments are exemplary. Although the specification may refer to "an", "one", or "some" embodiment(s) in several locations of the text, this does not necessarily mean that each reference is made to the same embodiment(s), or that a particular feature only applies to a single embodiment. Single features of different embodiments may also be combined to provide other embodiments. Furthermore, words "comprising" and "including" should be understood as not limiting the described embodiments to consist of only those features that have been mentioned and such embodiments may contain also features/structures that have not been specifically mentioned.

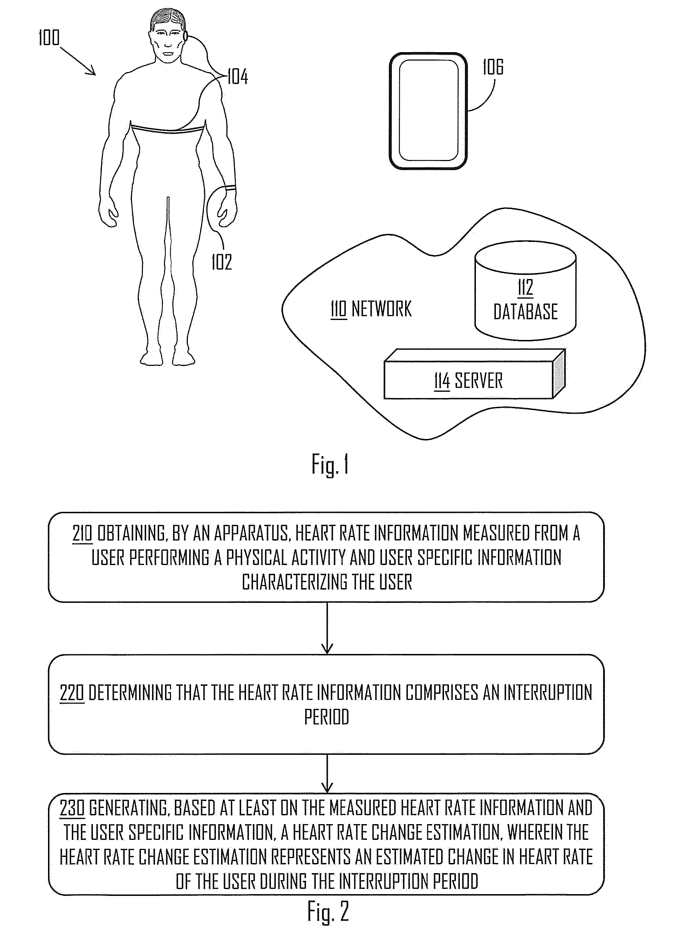

Figure 1 illustrates a heart activity measurement system to which embodiments of the invention may be applied. Referring to Figure 1, heart activity of a user 100 may be monitored by the user 100 using an activity tracker apparatus 102. The activity tracker apparatus 102 may be a portable or wearable electronic device, such as a wrist device 102. The wrist device 102 may comprise a heart activity circuitry configured to determine user's 100 heart activity, such as heart rate for example. The heart activity circuitry may comprise an optical heart activity sensor, such as a PPG (photoplethysmography) sensor, configured to measure user's 100 heart activity. The optical heart activity sensor may detect the user's 100 heart activity by optical heart rate measurement, which may comprise sending a light beam towards user's 100 skin and measuring the bounced and/or emitted light from the user's 100 skin. The light beam may alter when travelling through the user's 100 veins and the alterations may be detected by the optical heart rate activity sensor. By using the detected data, the wrist device 102, may determine user's 100 heart activity, such as heart rate for example.

The heart activity circuitry may comprise a bioimpedance sensor, wherein the bioimpedance sensor is configured to measure user's 100 heart activity. The bioimpedance measurement may be based on transmitting a radio signal into user's 100 skin, and observing changes in the radio signal due to impedance changes caused by, for example, blood volume changes. Thus, the user's 100 heart activity, such as heart rate, may be determined by the heart activity circuitry from the data produced by the bioimpedance sensor.

Further, besides these types of heart activity sensors, also other types of biosignal measurement sensors may be embedded into the heart activity circuitry. These types include but are not limited to the following: a Laser Doppler-based blood flow sensor, a magnetic blood flow sensor, an Electromechanical Film (EMFi) pulse sensor, a polarization blood flow sensor.

In an embodiment, the wrist device 102 comprises a motion sensor configured to measure motion induced by the user 100 to the wrist device 102 by moving hand in which the user 100 wears the wrist device 102. The motion sensor may use other motion data, such as location data of the user, to determine user's 100 motion.

In an embodiment, the motion sensor(s) comprise at least one of the following: an accelerometer, a magnetometer, and a gyroscope.

In an embodiment, the motion sensor comprises an accelerometer and a gyroscope. The motion sensor may further comprise sensor fusion software for combining the accelerometer data and gyroscope data so as to provide physical quantities, such as acceleration data, velocity data, or limb trajectory data in a reference coordinate system having orientation defined by a predetermined gyroscope orientation.

In an embodiment, the motion sensor comprises a gyroscope and a magnetometer. The motion sensor may further comprise sensor fusion software to combine gyroscope data and magnetometer data so as to provide a reference coordinate system for the gyroscope based on the Earth magnetic field measured by the magnetometer. In general, the sensor fusion software described above may combine measurement data acquired from at least two motion sensors such that measurement data acquired from one motion sensor is used to establish the reference coordinate system for the measurement data acquired from at least one other motion sensor.

Still referring to Figure 1, the heart activity measurement system may further comprise the external sensor devices 104 used by the user 100. The external sensor devices 104 may comprise sensors, such as a heart rate transmitter, a stride sensor, a positioning sensor, a cadence sensor and a power sensor, to mention a few. The heart rate transmitter may comprise at least one electrical, optical and/or bioimpedance sensor to measure user's 100 heart activity. The electrical sensor(s) may be, for example, based on Electrocardiography (EKG) measurement. The positioning sensor may comprise a GPS, a magnetometer and/or a Bluetooth sensor. Thus, the positioning may be based on, for example, GPS location and/or Bluetooth location. The magnetometer may provide direction data based on magnetic fields on earth and/or inside structures.

The external sensor devices 104 may comprise a head sensor, wherein the head sensor may be configured to measure heart activity of the user 100. The head sensor may be an ear sensor which may be placed within the user's 100 ear. The placement may be similar to placing earplug headphones. The head sensor may utilize optical measurement and/or bioimpedance measurement for the heart rate measurement, for example.

In an embodiment, the ear sensor is an in-ear sensor.

The external sensor devices 104 may transmit the sensor data to the wrist device 102, to a portable electronic device 106 and/or to a server 114, residing in a network 110, of the heart activity measurement system. The portable electronic device 106 may be a mobile phone, a smart phone, a palm device, a tablet computer, phablet or a portable digital assistant, for example. The wrist device 102 the portable electronic device 106 and/or the server 114 may receive the sensor data. Similarly, the wrist device 102 may transmit the heart activity data, provided by the heart activity circuitry, and/or the motion sensor data, provided by the motion sensor, to the portable electronic device 106 and/or the server 114. The wrist device 102, the portable electronic device 106 and/or the server 114 may comprise at least one processor configured to process the received external sensor data, the heart activity data and/or the motion data into a set of metrics describing the user's 100 physical activity, such as heart rate of the user 100.

The external sensor devices 104, the wrist device 102, the portable electronic device 106 and/or the server 114 may further comprise a communication circuitry, such as wireless communication circuitry, configured to enable sensor data transfer between the external sensor devices 104, wrist device 102, portable electronic device 106 and/or the server 114.

In an embodiment, the external sensor devices 104 comprise at least one external sensor device.

Further, the wrist device 102 and/or the portable electronic device 106 may comprise a memory, wherein the memory may be used by the devices to store the data from different sensor devices. The server 114 may use a database 112, such as a training database, to store the said data. The database 112 may reside in the network 110.

In an embodiment, the external sensor devices 104 are comprised in the wrist device 102.

In an embodiment, the wrist device 102 further comprises at least one of the following sensors: a temperature sensor, a positioning sensor and a pressure sensor. The positioning sensor may utilize GPS and/or Bluetooth information for locating the user 100. Further, the positioning sensor may comprise a magnetometer.

The described heart activity measurement system may enable the user to track the heart activity from different devices. For example, the wrist device 102 may be used during a physical exercise, wherein the heart activity information may be stored to the memory of the wrist device 102. The heart activity information may be transferred and stored to the portable electronic device 106 and/or to the server 114, where it may be viewed during the exercise and/or after the exercise. Further, the database 112 may comprise information from other users. This information may be usable by the user 100 in order to, for example, benchmark user's 100 physical progress.

However, the heart activity information, provided by the external sensor devices 104 and/or the heart activity circuitry of the wrist device 102, may in occasions be lacking. For example, the sensor(s) of the heart activity circuitry may not always work as planned, and thus the heart activity information may be lacking for a certain time period during the physical exercise. The measurement of the heart activity information may be interrupted, for example, by a faulty connection between user's 100 skin and the optical heart activity sensor. These interruptions may be quite annoying for the user 100, as the physical progress of the user 100 may then be more difficult to observe.

There is provided a solution for heart rate change estimation for a user 100 performing the physical exercise. The provided solution may enhance accuracy of the heart rate information by providing heart rate change estimation for at least some time periods, wherein the actual heart rate measurement may be interrupted.

Figure 2 illustrates a flow diagram according to an embodiment of the invention. Referring to Figure 2, in step 210, an apparatus, such as the wrist device 102, the external sensor devices 104, the portable electronic device 106 and/or the server 114, obtains heart rate information measured from the user 100 performing a physical activity and user specific information characterizing the user 100. The heart rate information may be obtained from the external sensor devices 104 and/or the heart activity circuitry of the wrist device 102. It may also be possible to obtain the heart rate information from the network 110, and more specifically from the database 112, as the heart rate information may be stored to the database 112.

In step 220, the apparatus determines that the heart rate information comprises an interruption period. This may mean that the heart rate information is lacking for the duration of the interruption period. It may further mean that the heart rate information is incomplete and/or unreliable during the interruption period. This may mean that even though the heart rate information may be available during the interruption period, it may be determined, by the apparatus, to be inaccurate.

In step 230, the apparatus generates, based at least on the measured heart rate information and the user specific information, a heart rate change estimation, wherein the heart rate change estimation represents an estimated change in heart rate of the user during the interruption period. For example, if the heart rate information comprises two heart rate values and the interruption period is detected between the said values, the apparatus may use the user specific information, such as exertion history characteristics to the user 100 and/or maximum heart rate value of the user 100, to generate the heart rate change estimation between the said values.

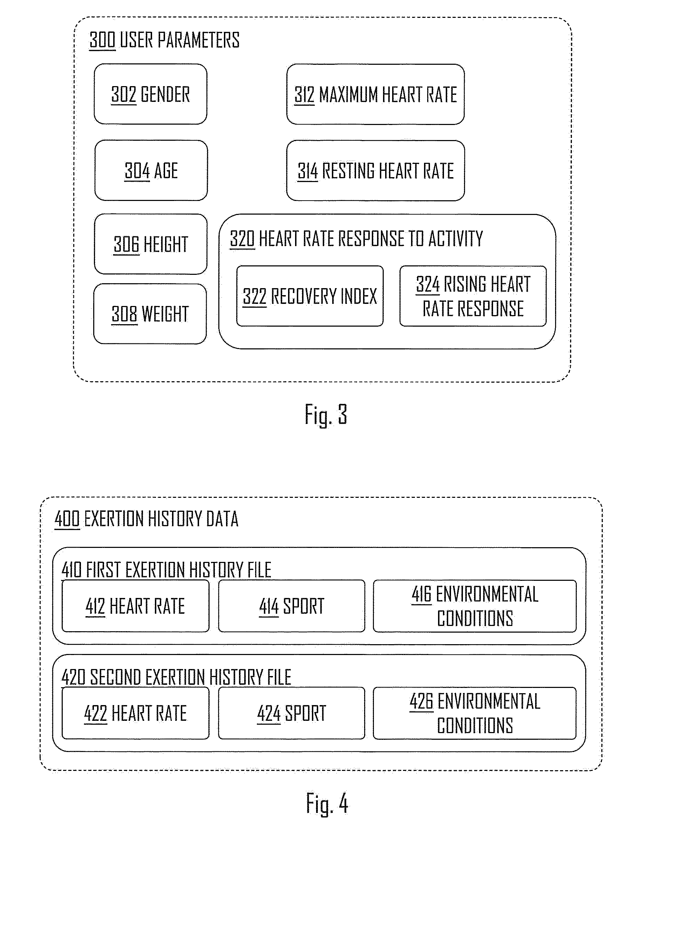

Let us now look closer on some embodiments of the invention. Figure 3 illustrates user parameters according to an embodiment of the invention. The user specific information of Figure 2 may comprise user parameters 300 that may be used by the apparatus to generate the heart rate change estimation as in step 230 of Figure 2. The user parameters 300 may comprise at least one physiological attribute of the user 100. These physiological attributes may comprise gender 302, age 304, height 306, weight 308, maximum heart rate 312, resting heart rate 314 and heart rate response to activity 320, to name a few. Further, the heart rate response to activity 320 may comprise recovery index 322 and rising heart rate response 324.

The maximum heart rate 312 may depend on, for example, the gender 302, the age 304 and overall physical condition of the user 100, but it may also be revealed by performing a physical exercise with maximum effort. The maximum heart rate 312 may describe the heart rate value which may be achieved by the user 100. Similarly, the resting heart rate 314 may depend on the user's 100 gender 302, age 304 and overall physical condition. Further, the resting hear rate 314 may be determined by measuring user's 100 heart rate during a resting period, for example, before getting up from a bed after a night of sleep.

The heart rate response to activity 320 may describe the user's 100 ability to react to changes in physical activity load when performing the physical activity. The recovery index 322 may describe the user's 100 ability to recover from the physical load when the physical load decreases. Thus, it may describe heart rate's rate of change, and more specifically how fast the heart rate decreases when the physical load decreases. The amount of physical load may affect the said rate of change. The said rate of change may be higher when the user's 100 heart rate is closer to the maximum heart rate 312. The said rate of change may decrease when the heart rate decreases.

Similarly, the rising heart rate response 324 may describe heart rate's rate of change when the physical load increases. The said rate of change may be higher when the user's 100 heart rate is closer to the resting heart rate 312. The said rate of change may decrease when the heart rate increases.

Further, the user's 100 recovery from the physical activity may become slower as the physical activity load increases. For example, if the user's 100 heart rate is close to maximum and the physical activity starts to decrease, it may be determined by the apparatus that the user's 100 heart rate decreases slower compared to a situation when the user's 100 heart rate starts to decrease from 80 % of maximum.

Figure 4 illustrates exertion history data according to an embodiment of the invention. Referring to Figure 4, the user specific information of Figure 2 may comprise exertion history data 400. The exertion history data 400 may be characteristics to the user 100 and/or the user characterized by the user parameters 300. The heart rate change estimation may be further based on the exertion history data 400. For example, the exertion history data 400 may be used to reveal user's 100 maximum heart rate and/or the heart rate response to activity 320. Thus, the heart rate change estimation may become more accurate, as it may be based on actual measurements from the exertion history data 400.

The exertion history data 400 may comprise at least some exertion history data of the user 100. The apparatus of Figure 2 may store information about physical exercises of the user 100 to the exertion history data 400, and use it later for the heart rate estimation. The exertion history data 400 may be stored in the apparatus's memory and/or in the database 112.

In an embodiment, the exertion history data 400 comprises exertion history data of at least one other user, and wherein the exertion history data of the at least one other user is obtained from a training database, such as the database 112 of Figure 1. Thus, the exertion history data 400 may comprise both exertion history data of the user 100 and exertion history data of the at least one other user, wherein the at least one other user is substantially characterized by the user parameters 300. The at least one other user's user parameters may be close to the user parameters 300 with a certain margin for error. For example, the user parameters may be similar with 90 % accuracy.

In embodiment, the user parameters between the user 100 and the at least one other user are similar at least with 95 % accuracy.

In embodiment, the user parameters between the user 100 and the at least one other user are similar at least with 99 % accuracy.

In an embodiment, the exertion history data 400 comprises only the exertion history data of the at least one other user.

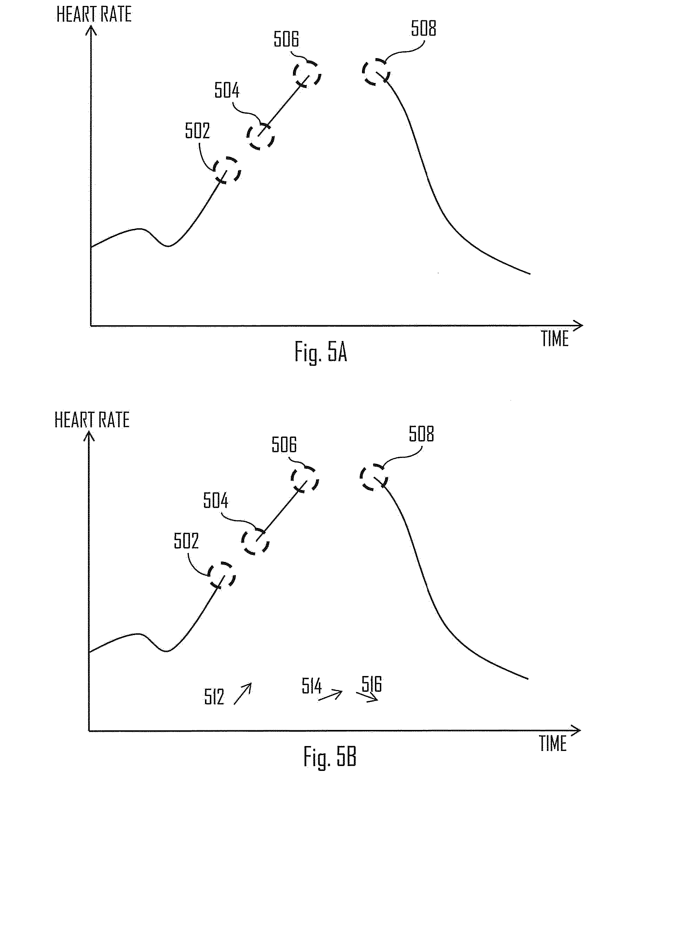

As the database 112 may provide the exertion history data of the at least one other user, the efficiency of the heart rate estimation may increase as there may be more references for the heart rate estimation to suit different training scenarios and situations. These different situations are discussed later with reference to Figures 5A to 5E.

Still referring to Figure 4, the exertion history data 400 may comprise at least one exertion history file 410, 420 comprising at least one of the following: heart rate 412, 422 during the at least one exertion history file 410, 420, sport 414, 424 of the at least one exertion history file 410, 420, and environmental conditions 416, 426 of the at least one exertion history file 410, 420. The heart rate 412, 422 may illustrate the heart rate of a user, such as the user 100 or the at least one other user, during the physical exercise that the at least one exertion history file 410, 420 is describing. The heart rate 412, 422 may be illustrated with a function, a set of measurements and/or a diagram, to name a few. The heart rate 412 may comprise actual measurement results and/or estimations of the heart rate. The parts which are actual measurements and which are estimations may be indicated with different indicators. Thus, the apparatus may be aware which heart rate information may be more accurate.

The sport 414, 424 may describe the sport and/or activity performed by the user during the at least one exertion history file 410, 420. One file, such as the first exertion history file 410, may comprise one or more sports. This may happen if the at least one exertion history file 410, 420 describes a multisport activity, for example. Different heart rate 412, 422 periods may correspond to the one or more sports, and thus the apparatus may be aware that a certain heart rate period describes, for example, running.

The environmental conditions 416, 426 may comprise weather conditions and/or terrain details, for example. The environmental conditions 416, 426 may affect the physical load of the physical exercise. Therefore, they may be used to enhance the heart rate estimation. For example, if the user 100 is skiing on a flat terrain the physical load may be quite different than when the skiing is performed on a rugged terrain.

Let us now look closer on the generating the heart rate change estimation by the apparatus. Figure 5A illustrates an embodiment of the invention. Referring to Figure 5A, the user's 100 heart rate may be illustrated in a function of time. The illustrated heart rate may be the measured heart rate described in Figure 2. As in step 220 of Figure 2, the apparatus may detect and/or determine that the illustrated heart rate information comprises interruption periods, which may be seen between the points 502, 504, and between points 506, 508. The points 502, 504, 506, 508 may be heart rate measurement results. For example, the heart rate measurement 506 may indicate that the user's 100 heart rate in that specific time is 160 beats per minute (bpm).

The interruption determination may be performed during the physical exercise and/or after the physical exercise. The determination may be based, at least partially, on the measuring interval between the heart rate measurements. For example, the heart rate may normally be measured once per second, and if the interval between the heart rate measurements is more than one second, the apparatus may determine that there is an interruption in the heart rate measurement.

In an embodiment, the interval between the heart rate measurements is at least 10 seconds before the apparatus determines that there is an interruption period in the heart rate measurement.

In an embodiment, the measured heart rate information comprises at least one heart rate measurement 502, 504, 506, 508. The heart rate change estimation may be thus based on only one heart rate measurement 502, 504, 506, 508. For example, if the maximum heart rate is know it may be possible to generate the change estimation between the measured heart rate value 502, 504, 506, 508 and the maximum heart rate. In order to do this, the exertion history data 400 may be used. It may also be possible to utilize physical activity metric for the change estimation. This is discussed later in more detail.

In an embodiment, the measured heart rate information comprises at least two heart rate measurements 502, 504, 506, 508, and wherein the interruption period is determined to be between the said at least two heart rate measurements 502, 504, 506, 508.

Figure 5B illustrates an embodiment of the invention. Referring to Figure 5B, the heart rate change estimation may indicate the amount that heart rate changes in a certain time. For example, when the heart rate is lower and exhaustion of the physical exercise is increasing and/or staying the same, the heart rate may increase quite fast as indicated by the arrow 512. Thus, the heart rate change estimation during the interruption period between the points 502, 504 may be indicated by the arrow 512.

As the physical exercise becomes more exhausting, the user's 100 heart rate may reach the maximum heart rate 312. Based on the user specific information and/or the maximum heart rate 312, as described above, the apparatus may generate, between the points 506, 508, the heart rate change estimation comprising arrows 514, 516. As indicated by the arrows 514, 516, the heart rate change may now be estimated to be slower compared to the arrow 512.

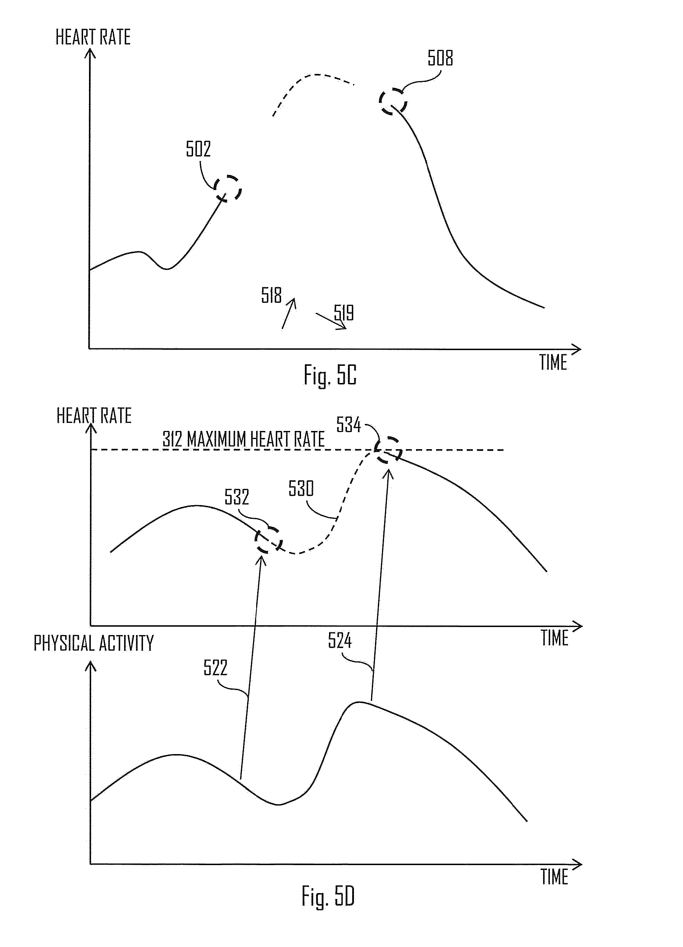

Figure 5C illustrates an embodiment of the invention. Referring to Figure 5C, the heart rate change estimation may be generated between the points 502, 508. However, the heart rate change estimation may not necessarily cover all of the time between the points 502, 508. This may mean that the heart rate change estimation is generated within the interruption period, but not necessarily covering it all. Further, it may also be possible that the interruption period is determined to be something less than the time between the points 502, 508.

Arrows 518, 519 may illustrate the heart rate change estimation during the interruption period. The heart rate change estimation may comprise an estimated heart rate shown in Figure 5C with a dash curve.

In an embodiment, the apparatus generates a heart rate estimation based on the heart rate change estimation, wherein the heart rate estimation illustrates the estimated heart rate of the user 100 during the interruption period.

Figure 5D illustrates and embodiment of the invention. Referring to Figure 5D, the apparatus may determine an interruption period in the heart rate information between heart rate measurements 532, 534. The apparatus may obtain physical activity information of the user during the physical activity. Thus, the generating the heart rate change estimation may be further based on the physical activity information. For example, the physical activity information and/or motion activity information may be used to determine whether the heart rate is increasing, decreasing or staying the same and at what speed.

The physical activity information may be produced by the motion sensor described in relation to Figure 1, for example. The producing of the physical activity information may have less lag than the measured heart rate information as it may require less processing and/or modulating than the measured heart rate information. Therefore, the physical activity information may be already available for the whole and/or partial duration of the interruption period, when the interruption is detected. In Figure 5D, the arrow 522 illustrates that the physical activity information, corresponding to the heart rate measurement 532, may be available for the apparatus earlier than the heart rate measurement 532. Thus, the heart rate measurement 532 and the corresponding physical activity information may have been measured at the same time. Same may be true for the heart rate measurement 534 and the corresponding physical activity information shown with an arrow 524.

Dash curve 530 may illustrate the heart rate change estimation. The dash curve 530 may therefore indicate the estimated changes in the heart rate of the user 100 during the interruption period. The dash curve 530 may substantially follow alterations of the physical activity diagram. Further, the maximum heart rate 312 may act as a upper limit for the generation of heart rate change estimation.

In an embodiment, the physical activity information is used, by the apparatus, as reference, wherein the heart rate change estimation is generated based on the user specific information, and wherein the generated heart rate change estimation is validated against the physical activity information. Therefore, the physical activity information may provide further knowledge about the accuracy of the heart rate change estimation.

In an embodiment, the apparatus determines, based on the motion activity information provided by the motion sensor(s) that the physical exercise load increases. The apparatus may then determine, based on the user specific information and the motion activity information, the heart rate change estimation during the interruption period. The apparatus may further determine, based on the heart rate change estimation, that the user's 100 heart rate is over the maximum heart rate 312 and alter and/or decrease the heart rate change estimation to be in line with the maximum heart rate 312.

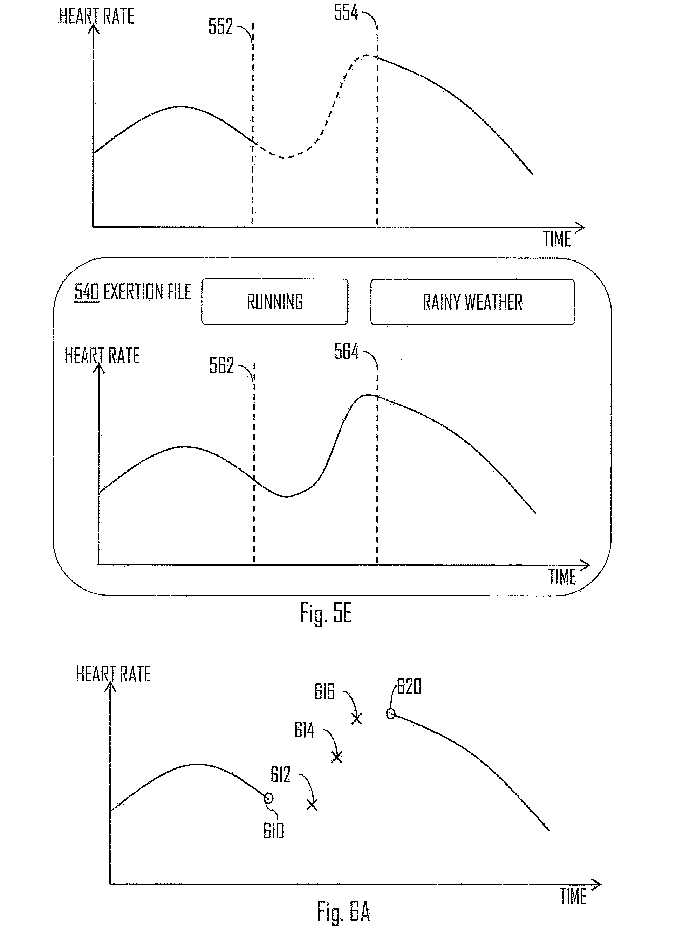

Figure 5E illustrates an embodiment of the invention. Referring to Figure 5E, the apparatus may detect the interruption period in the heart rate information between the lines 552, 554. The apparatus may use the exertion history data 400 of Figure 4 to find a matching exercise to the current exercise. The apparatus may find a matching exertion file 540 which may at least partly match to the current exercise.

In an embodiment, the matching is based on the measured heart rate information. Thus, the apparatus may compare the exertion file's 540 heart rate information to the heart rate information of the current exercise on the parts where there is reliable heart rate information available. Therefore, the interruption period, indicated between lines 552, 554, may be left outside the comparison.

The exertion file 540 may be the at least one exertion history file 410, 420 of Figure 4, for example. The exertion file 540 may comprise heart rate information, sport and environmental conditions of the exercise performed. Thus, running and rainy weather may be used to help to find the matching file for the current exercise and/or a part of the exercise.

The apparatus may use the heart rate information of the exertion file 540 between the lines 562, 564 to generate the heart rate change estimation for the current exercise for the duration of the interruption period that was determined to be between the lines 552, 554. Naturally, the exertion file 540 may comprise activity information which may be used in the generation of the heart rate change estimation.

In an embodiment, the heart rate change estimation comprises a heart rate function, wherein the heart rate function represents estimated changes in heart rate of the user during the interruption period as a function of time.

Figure 6A illustrates an embodiment of the invention. Referring to Figure 6A, the heart rate change estimation during the interruption period, between points 610, 620, may comprise at least one estimated heart rate value 612, 614, 616. The heart rate change estimation may thus be indicated by the at least one estimated heart rate value 612, 614, 616. The points 610, 620 may be heart rate measurements that are comprised in the measured heart rate information. Therefore, the at least one estimated heart rate value 612, 614, 616 may be located between the heart rate measurements 610, 620.

In an embodiment, it is possible to indicate the estimated changes in the heart rate between the points 610, 612, 614, 616, 620. From the said indication, the actual points 612, 614, 616 may be determined by the apparatus.

In an embodiment, the heart rate change estimation comprises the heart rate function and the at least one estimated heart rate value 612, 614, 616.

Still referring to Figure 6A, the estimated heart rate values 612, 614, 616 may be generated by the apparatus based on the heart rate measurements 610, 620 and the user specific information, as described earlier. The apparatus may, for example, use the rising heart rate response 324 to determine rising rate of the heart rate between the measurements 610, 620. Further, the apparatus may use the recovery index 322 and, for example, physical activity information to determine that the estimated heart rate value 612 is below the heart rate measurement 610.

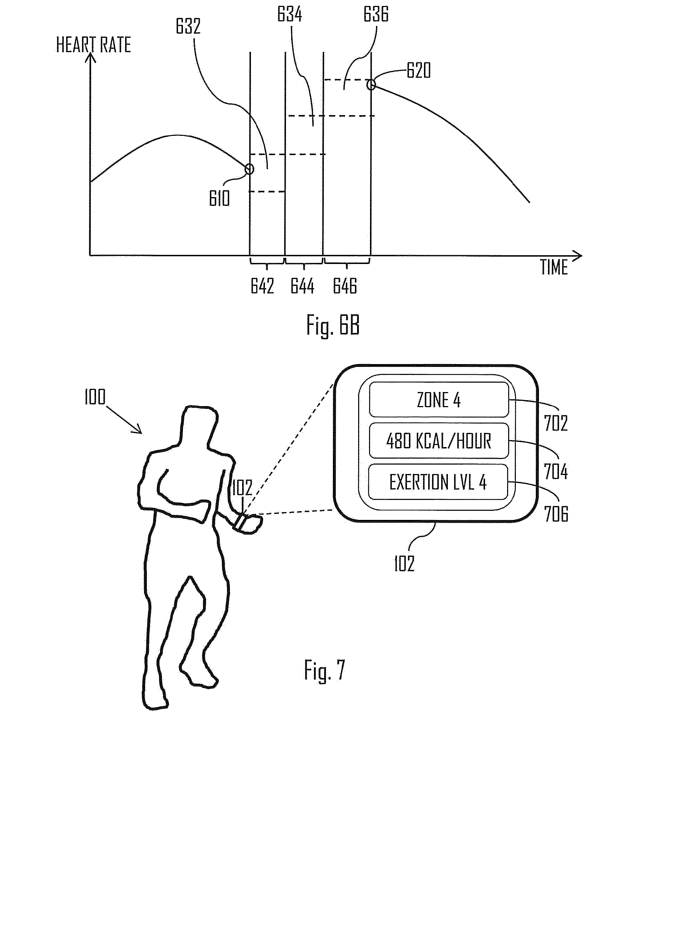

Figure 6B illustrates an embodiment of the invention. Referring to Figure 6B, the heart rate change estimation may comprise a heart rate zone 632, 634, 636, wherein the estimated changes in heart rate of the user during the interruption period, indicated between the points 610, 620, may be represented with the heart rate zone 632, 634, 636. The heart rate zones 632, 634, 636 may comprise an upper and lower boundaries indicating the heart rate zones 632, 634, 636 between the said boundaries. The upper and lower boundaries may be indicated with dash lines in Figure 6B.

In an embodiment, the heart rate zones 632, 634, 636 are at least partially over each other. This may mean that a certain heart rate value may be comprised in at least two heart rate zones.

In an embodiment, the heart rate change estimation during the interruption period, indicated between the points 610, 620, is illustrated with three heart rate zones 632, 634, 636. The first heart rate zone 642 may illustrate the heart rate change estimation during a first timeframe indicated with a bracket 642. The second heart rate zone 644 may illustrate the heart rate change estimation during a second timeframe indicated with a bracket 644. The third heart rate zone 646 may illustrate the heart rate change estimation during a third timeframe indicated with a bracket 646. Therefore, the heart rate zones 632, 634, 636 may indicate the estimated boundaries for heart rate changes during the interruption period.

Indicating heart rate change estimation with the heart rate zones may be beneficial, as it may not always be possible to generate, by the apparatus, more accurate estimation of changes in the heart rate. Thus, it may be beneficial to indicate that the user's 100 heart rate change is within a certain zone(s) during the interruption period. This way the indicated heart rate change estimation may be more realistic, and thus more usable for observing the physical progress of the user. It may also be possible that the heart rate change estimation comprises exact information about the heart rate and the heart rate zone(s). Therefore, the heart rate change estimation may comprise heart rate zone(s), heart rate function(s) and/or estimated heart rate value(s).

Figure 7 illustrates an embodiment of the invention. Referring to Figure 7, the apparatus of Figure 2, such as the wrist device 102, may generate energy expenditure information 704 and/or an exertion parameter 706 based on the heart rate change estimation 702, wherein the heart rate change estimation may be as described above.

The energy expenditure information 704 may illustrate the energy used by the user 100 at a certain moment of the physical exercise and/or cumulative energy used during at least part of the physical exercise. Similarly, the exertion parameter 706 may illustrate effectiveness of the physical exercise at the certain moment of the physical exercise and/or it may illustrate the effectiveness of at least part of the physical exercise.

In an embodiment, the exertion parameter is illustrated with a value ranging from 1 to 5. Value 1 may mean that the physical load is low and value 5 may mean that the physical load is the high.

In an embodiment, the apparatus displays the heart rate change estimation 702, energy expenditure information 704 and/or the exertion parameter 706 to the user 100 during the physical exercise. Thus, the user 100 may benefit from the heart rate change estimation during the exercise.

In an embodiment, the apparatus generates and/or updates, after generating the heart rate change estimation 702, at least one of the following: the energy expenditure information 704 and the exertion parameter 706.

In an embodiment, the apparatus determines that the physical exercise has ended, and generates and/or updates at least one of the following: heart rate change estimation 702, the energy expenditure information 704 and the exertion parameter 706. Thus, the apparatus may generate and/or update the said information retrospectively. This may be beneficial as the exertion files may be updated to be more accurate after the physical exercise.

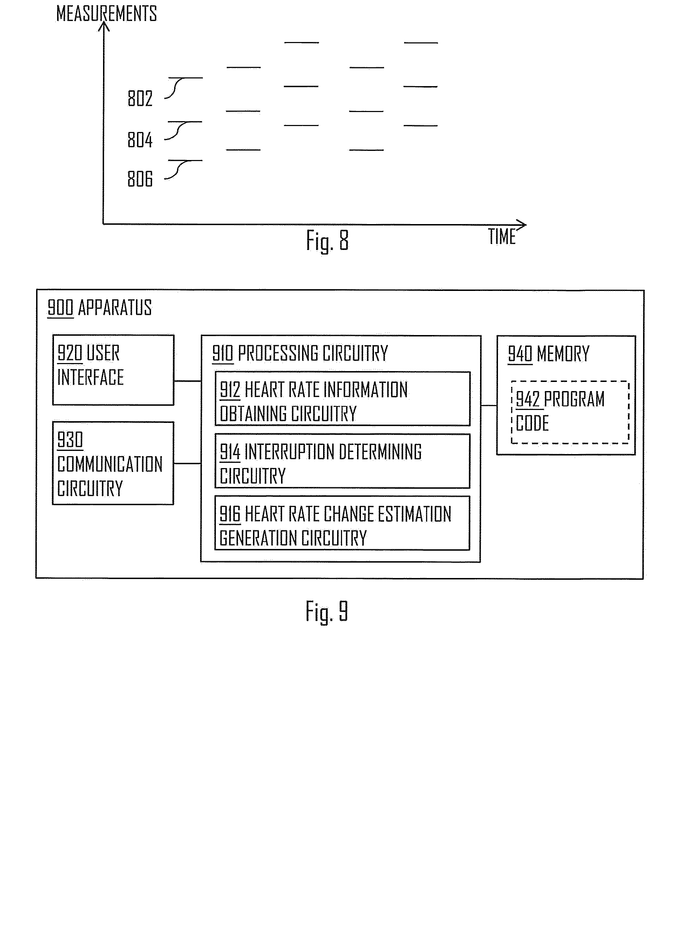

Figure 8 illustrates an embodiment of the invention. Referring to Figure 8, the heart activity circuitry, motion sensor and/or the external sensor devices 104 described in Figure 1 may produce measurements illustrated with lines 802, 804, 806. Each measurement may comprise, for example, three lines, wherein the first line 802 may indicate measured heart rate, the second line 804 may indicate motion error and the third line 806 may indicate motion of the user 100, such as steps per minute. These measurements may be processed, by the apparatus, to a clearer form. For example, the heart rate measurements 802 may be illustrated as shown in previous Figures.

However, if the heart rate measurement is corrupted and/or interrupted, the apparatus may misinterpret the lines 802, 804, 806. For example, the apparatus may determine that line 806 is illustrating the heart rate. To prevent this, the measured heart rate information, user specific information and/or physical activity information may be used to find the correct value. For example, the apparatus may determine, based on the physical activity information that the line 806 represents the motion of the user. Thus, the apparatus may then know that the heart rate measurement is not available for that certain time, and use the heart rate information, user specific information and/or physical activity information to generate the heart rate change estimation.

In an embodiment, the apparatus uses the heart rate information, user specific information and/or physical activity information to validate that the line 802 is the correct heart rate measurement.

In an embodiment, the apparatus generates the heart rate change estimation as described above, and uses the heart rate change estimation to find the correct value amongst the measurements 802, 804, 806 for the heart rate of the user 100. Therefore, the heart rate change estimation may be used to find and/or validate the exact heart rate measurement amongst the measurements 802, 804, 806. In order to achieve this, the heart rate change estimation may indicate what the heart rate should be at a certain time. Using this information, the apparatus may determine which of the measurements 802, 804, 806 may be closest to the estimated heart rate, and use that measurement 802, 804, 806 as the heart rate value. This may enhance the reliability of the measurement, as sometimes the exact value may be measured, but not indicate properly.

In an embodiment, the heart rate change estimation comprises at least one heart rate zone, wherein the estimated change in heart rate of the user during the interruption period is represented with the at least one heart rate zone, and the apparatus searches a measured heart rate value within the at least one heart rate zone. Thus, the at least one heart rate zone may be used to find the correct measurement.

In an embodiment, the heart rate change estimation comprises at least one estimated heart rate value.

In an embodiment, the measured heart rate information comprises at least two heart rate measurements, and wherein the interruption period is determined to be between the said at least two heart rate measurements.

In an embodiment, the user specific information comprises exertion history data that is characteristics to the user characterized by the user parameters, and wherein the generating of the heart rate change estimation is further based on the exertion history data.

In an embodiment, the exertion history data comprises at least some exertion history data of the user.

In an embodiment, the exertion history data comprises exertion history data of at least one other user, and wherein the exertion history data of the at least one other user is obtained from a training database.

In an embodiment, the exertion history data comprises at least one exertion history file comprising at least one of the following: heart rate during the at least one exertion history file, sport of the at least one exertion history file and environmental conditions of the at least one exertion history file.

In an embodiment, the apparatus is further configured to reconstruct a heart rate curve on the basis of the heart rate change estimation, wherein the heart rate curve represents changes in heart rate during the interruption period.

In an embodiment, the apparatus is further configured to generate energy expenditure information based on the heart rate change estimation.

In an embodiment, the apparatus is further configured to generate an exertion parameter based on the heart rate change estimation, wherein the exertion parameter illustrates effectiveness of the physical exercise.

In an embodiment, the apparatus is further configured to display at least one of the following during the physical exercise: the energy expenditure information and the exertion parameter.

Figure 9 illustrates a block diagram of the apparatus according to an embodiment of the invention. Referring to Figure 9, the apparatus 900 may be the apparatus performing the steps 210, 220, 230 of Figure 2.

In an embodiment, the apparatus 900 is and/or is comprised in the wrist device 102, the portable electronic device 106, the server 114 and/or the external sensor device(s) 104.

In an embodiment, the apparatus 900 is configured to perform functions of multiple devices 102, 106, 114, 104. This may mean, for example, that the apparatus 900 may receive information, such as the heart rate information, from external source and use another apparatus to process the heart rate information into the heart rate change estimation. The apparatus 900 may then receive the heart rate change estimation, and display it to the user 100. For example, the external sensor device(s) 104 may provide the heart rate information and send it to the wrist device 102. The wrist device 102 may receive the information and transmit it to the server 114 for processing. The server 114 may process determine the interruption period, generate the heart rate change estimation and send the heart rate change estimation back to the wrist device 102. The wrist device 102 may receive the heart rate change estimation and display it to the user. Thus, the described solution may be applicable, at least partially, to each of the devices 102, 106, 114, 104.

Still referring to Figure 9, the apparatus may comprise a processing circuitry 910, such as at least one processor, and at least one memory 940, including a computer program code (software) 942, wherein the at least one memory 940 and the computer program code (software) 942, are configured, with the at least one processor, to cause the respective apparatus 900 to carry out any one of the embodiments of Figures 1 to 8, or operations thereof.

The memory 940 may be implemented using any suitable data storage technology, such as semiconductor based memory devices, flash memory, magnetic memory devices and systems, optical memory devices and systems, fixed memory and removable memory. The memory 940 may be used to store information, such as exertion file(s), user specific information, measured heart rate information and heart rate change estimation, for example.

The apparatus 900 may further comprise a communication circuitry 930 comprising hardware and/or software for realizing communication connectivity according to one or more communication protocols. The communication circuitry 930 may provide the apparatus 900 with communication capabilities to exchange data between devices. The communication circuitry 930 may enable wireless connection. The wireless communication circuitry 930 may be based on Bluetooth® specifications, e.g. Bluetooth Low Energy, Near-Field-Communication (NFC) technology, wherein the NFC technology may enable data transfer on short distances, and/or Wireless Local Area Access (WLAN). However, the wireless communication circuitry 930 may not be limited to these technologies, and thus, for example, Long Term Evolution (LTE) communication link, or similar, may also be provided by the communication circuitry 930, providing the apparatus 900 a long range communication capability.

The apparatus 900 may also comprise user interface 920 comprising, for example, at least one keypad, a microphone, a touch display, a display and a speaker. The user interface 920 may be used to control the respective apparatus by the user 100.

The processing circuitry 910 may comprise a heart rate information obtaining circuitry 912 configured to obtain heart rate information measured from the user 100 performing a physical activity and user specific information characterizing the user 100. The communication circuitry 930 may be used to receive the data from, for example, external sensor device(s) 104 and/or from the sensor(s) of the wrist device 102.

The processing circuitry 910 may further comprise an interruption determining circuitry 914 configured to determine that the heart rate information, obtained by the heart rate information obtaining circuitry 912, comprises an interruption period.

Further, the processing circuitry 910 may comprise a heart rate change estimation generation circuitry 916 configured to generate, based at least on the measured heart rate information and the user parameters, a heart rate change estimation, wherein the heart rate change estimation represents an estimated change in heart rate of the user during the interruption period.

In an embodiment, the apparatus 900 generates the heart rate change estimation with data received from external source(s).

In an embodiment, the apparatus 900 is capable of providing the data for the heart rate change estimation. Therefore, external device(s) and/or sensor(s) may not be required.

In an embodiment, the apparatus 900 comprises the heart activity circuitry and/or the motion sensor(s) of Figure 1.

As used in this application, the term 'circuitry' refers to all of the following: (a) hardware-only circuit implementations, such as implementations in only analog and/or digital circuitry, and (b) combinations of circuits and soft-ware (and/or firmware), such as (as applicable): (i) a combination of processor(s) or (ii) portions of processor(s)/software including digital signal processor(s), software, and memory(ies) that work together to cause an apparatus to perform various functions, and (c) circuits, such as a microprocessor(s) or a portion of a microprocessor(s), that require software or firmware for operation, even if the software or firmware is not physically present. This definition of 'circuitry' applies to all uses of this term in this application. As a further example, as used in this application, the term 'circuitry' would also cover an implementation of merely a processor (or multiple processors) or a portion of a processor and its (or their) accompanying software and/or firmware. The term 'circuitry' would also cover, for example and if applicable to the particular element, a baseband integrated circuit or applications processor integrated circuit for a mobile phone or a similar integrated circuit in a server, a cellular network device, or another network device.

In an embodiment, at least some of the processes described in connection with Figures 1 to 8 may be carried out by an apparatus comprising corresponding means for carrying out at least some of the described processes. Some example means for carrying out the processes may include at least one of the following: detector, processor (including dual-core and multiple-core processors), digital signal processor, controller, receiver, transmitter, encoder, decoder, memory, RAM, ROM, software, firmware, display, user interface, display circuitry, user interface circuitry, user interface software, display software, circuit, antenna, antenna circuitry, and circuitry. In an embodiment, the at least one processor, the memory, and the computer program code form processing means or comprises one or more computer program code portions for carrying out one or more operations according to any one of the embodiments of Figures 1 to 8 or operations thereof.

According to yet another embodiment, the apparatus carrying out the embodiments comprises a circuitry including at least one processor and at least one memory including computer program code. When activated, the circuitry causes the apparatus to perform at least some of the functionalities according to any one of the embodiments of Figures 1 to 8, or operations thereof.

The techniques and methods described herein may be implemented by various means. For example, these techniques may be implemented in hardware (one or more devices), firmware (one or more devices), software (one or more modules), or combinations thereof. For a hardware implementation, the apparatus(es) of embodiments may be implemented within one or more application-specific integrated circuits (ASICs), digital signal processors (DSPs), digital signal processing devices (DSPDs), programmable logic devices (PLDs), field programmable gate arrays (FPGAs), processors, controllers, micro-controllers, microprocessors, other electronic units designed to perform the functions described herein, or a combination thereof. For firmware or software, the implementation can be carried out through modules of at least one chip set (e.g. procedures, functions, and so on) that perform the functions described herein. The software codes may be stored in a memory unit and executed by processors. The memory unit may be implemented within the processor or externally to the processor. In the latter case, it can be communicatively coupled to the processor via various means, as is known in the art. Additionally, the components of the systems described herein may be rearranged and/or complemented by additional components in order to facilitate the achievements of the various aspects, described with regard thereto, and they are not limited to the precise configurations set forth in the given figures, as will be appreciated by one skilled in the art.

Embodiments as described may also be carried out in the form of a computer process defined by a computer program. The computer program may be in source code form, object code form, or in some intermediate form, and it may be stored in some sort of carrier, which may be any entity or device capable of carrying the program. For example, the computer program may be stored on a computer program distribution medium readable by a computer or a processor. The computer program medium may be, for example but not limited to, a record medium, computer memory, read-only memory, electrical carrier signal, telecommunications signal, and software distribution package, for example. Coding of software for carrying out the embodiments as shown and described is well within the scope of a person of ordinary skill in the art.

Even though the invention has been described above with reference to an example according to the accompanying drawings, it is clear that the invention is not restricted thereto but can be modified in several ways within the scope of the appended claims. Therefore, all words and expressions should be interpreted broadly and they are intended to illustrate, not to restrict, the embodiment. It will be obvious to a person skilled in the art that, as technology advances, the inventive concept can be implemented in various ways. Further, it is clear to a person skilled in the art that the described embodiments may, but are not required to, be combined with other embodiments in various ways.

## Claims

1. A method comprising:
obtaining, by an apparatus, heart rate information measured from a user performing a physical activity and user specific information characterizing the user;
determining that the heart rate information comprises an interruption period; and
generating, based at least on the measured heart rate information and the user specific information, a heart rate change estimation, wherein the heart rate change estimation represents an estimated change in heart rate of the user during the interruption period.

2. The method of claim 1, wherein the user specific information comprises user parameters comprising at least one physiological attribute of the user.

3. The method of claim 2, wherein the user specific information comprises exertion history data that is characteristics to the user **characterized by** the user parameters, and wherein the generating of the heart rate change estimation is further based on the exertion history data.

4. The method of claim 3, wherein the exertion history data comprises at least some exertion history data of the user.

5. The method of any of claims 3 to 4, wherein the exertion history data comprises exertion history data of at least one other user, and wherein the exertion history data of the at least one other user is obtained from a training database.

6. The method of any of claims 3 to 5, wherein the exertion history data comprises at least one exertion history file comprising at least one of the following:
heart rate during the at least one exertion history file;
sport of the at least one exertion history file; and
environmental conditions of the at least one exertion history file.

7. The method of any preceding claim, further comprising:
obtaining physical activity information of the user during the physical activity, wherein the generating the heart rate change estimation is further based on the physical activity information.

8. The method of any preceding claim, wherein the heart rate change estimation comprises at least one heart rate zone, wherein the estimated change in heart rate of the user during the interruption period is represented with the at least one heart rate zone, the method further comprising:
searching a measured heart rate value within the at least one heart rate zone.

9. The method of any preceding claim, further comprising:
reconstructing a heart rate curve on the basis of the heart rate change estimation, wherein the heart rate curve represents changes in heart rate during the interruption period.

10. The method of any preceding claim, wherein the heart rate change estimation comprises at least one estimated heart rate value.

11. The method of any preceding claim, wherein the measured heart rate information comprises at least two heart rate measurements, and wherein the interruption period is determined to be between the said at least two heart rate measurements.

12. The method of any preceding claim, further comprising:
generating energy expenditure information based on the heart rate change estimation.

13. The method of any preceding claim, further comprising:
generating an exertion parameter based on the heart rate change estimation, wherein the exertion parameter illustrates effectiveness of the physical exercise.

14. The method of any of claims 12 to 13, further comprising:
displaying at least one of the following during the physical exercise: the energy expenditure information, and the exertion parameter.

15. An apparatus comprising means for performing the method according to any of claims 1 to 14.

16. A computer program product comprising program instructions when loaded into an apparatus execute the method according to any of claims 1 to 14.
